Europäisches Patentamt

European Patent Office

Office européen des brevets

(11) Veröffentlichungsnummer : **0 022 987**
**B1**

(12) **EUROPÄISCHE PATENTSCHRIFT**

(45) Veröffentlichungstag der Patentschrift :
20.04.83

(51) Int. Cl.³ : **A 61 M   5/315**

(21) Anmeldenummer : **80103948.8**

(22) Anmeldetag : **10.07.80**

(54) **Spritze.**

(30) Priorität : **23.07.79 US 59861**

(43) Veröffentlichungstag der Anmeldung :
**28.01.81 Patentblatt 81/04**

(45) Bekanntmachung des Hinweises auf die Patenterteilung : **20.04.83 Patentblatt 83/16**

(84) Benannte Vertragsstaaten :
**DE FR GB IT SE**

(56) Entgegenhaltungen :
**DE A 2 315 018**
**GB A 1 212 823**
**US A 3 563 240**

(73) Patentinhaber : **Cutter Laboratories, Inc.**
**Fourth and Parker Streets**
**Berkeley California 94710 (US)**

(72) Erfinder : **Travaient, Louis J.**
**510 Southwest Raintree Drive**
**Lee's Summit Missouri 64063 (US)**
Erfinder : **Arenson, Herbert**
**2121 Johnson Drive**
**Shawnee Mission Kansas 66205 (US)**

(74) Vertreter : **Dill, Erwin, Dr. et al**
**c/o BAYER AG Zentralbereich Patente Marken und Lizenzen Bayerwerk**
**D-5090 Leverkusen 1 (DE)**

Spritze

Die Erfindung betrifft Spritzen, insbesondere Kunststoffspritzen und vor allem solche, die zur Abgabe von zähen Arzneimittelzubereitungen bestimmt sind.

Bei der Herstellung vorgefüllter Spritzen treten Probleme auf, die bisher noch nicht zufriedenstellend gelöst werden konnten. Normalerweise wird das Ausstoßende der Spritze zunächst mit einer Kappe versehen, es wird das Medikament bis zu einer angemessenen Höhe in das Spritzengehäuse eingefüllt, und es wird sodann der Kolben eingeführt, bis die Kolbenspitze mit ihrer Dichtung das Medikament berührt. Die Luft in dem Spritzengehäuse oberhalb des Medikamentes muß während dieses Endschrittes auf irgendeine Weise ausgetrieben werden. Dies läßt sich entweder erreichen, indem ein Luftdurchlaß in der Kolbenspitzendichtung ausgebildet wird oder indem man während des Einsetzens des Kolbens von Hand einen Draht oder eine ähnliche Vorrichtung zwischen die Wand des Spritzengehäuses und die Dichtung legt, um einen Luftauslaß zu schaffen und dann den Draht wieder herauszieht. Das erste Vorgehen ist nicht zufriedenstellend, weil durch die Luftauslaßöffnung das Medikament austreten kann. Das zweite Vorgehen ist zeitaufwendig und erlaubt keine automatisierten Vorfülltechniken. Das letztgenannte Verfahren würde außerdem erfordern, daß die Dichtung auf der Kolbenspitze eine dünne flexible Wischergestaltung hat ; eine solche Dichtung unterliegt jedoch, insbesondere wenn sie aus Kunststoff hergestellt ist, schnell Verformungen, die eine ungenügende Abdichtung zur Folge hätten. Dichtungen aus Gummi sind häufig unerwünscht, weil das Medikament oder sein Bindemittel in ungünstiger Weise reagieren können.

Eine Anzahl von Spritzen für die abgemessene Dosierung eines Medikamentes sind in US-PS 2 764 981, 2 856 925, 2 869 541, 2 875 761 und 3 934 586 beschrieben, die zur Ermöglichung der Abgabe von abgemessenen Dosen verschiedene Anschlagmittel an dem Kolben zeigen. US-PS 3 563 240 und 4 153 056 veranschaulichen Spritzen, bei denen eine Feineinstellung abgemessener Dosierungen mit einem Ring erreicht wird, der auf die Kolbenstange aufgeschraubt ist.

In der GB-PS 1 212 823 werden Spritzen mit einem Spritzengehäuse beschrieben, an dem ein Ausstoßende und ein offenes, gegenüberliegendes Ende zur Aufnahme eines Kolbens ausgebildet sind, wobei zu dem Kolben eine Kolbenstange gehört, an deren einem Ende sich eine Griffplatte befindet und an deren anderem Ende ein Kolbenkopf mit einer daran befestigten abnehmbaren Kolbendichtung vorgesehen sind, die aus einem im wesentlichen rohrförmigen Körper mit einem vorderen und einem hinteren Ende besteht, dessen Außenfläche auf der Innenfläche des Spritzengehäuses verschiebbar ist und der auf seiner Innenseite Paßteile aufweist, die mit auf dem Kolbenkopf angeordneten Eingriffteilen zusammenwirken. Auch diese Spritze hat den Nachteil, daß die Kolbendichtung auf dem Kolbenkopf schwierig aufzusetzen ist und eingeschlossene Luft beim Einführen des Kolbens in das gefüllte Spritzengehäuse während der Herstellung der Spritze nicht entweichen kann.

Die erfindungsgemäße Spritze überwindet die erwähnten Schwierigkeiten und ist besonders geeignet für die automatisierte Vorfüllung von Wegwerfspritzen aus Kunststoff, insbesondere solchen Spritzen, die zähe Arzneimittelzubereitungen, wie Pasten o. dgl. enthalten.

Die erfindungsgemäße Spritze weist ein Spritzengehäuse mit einem Ausstoßende und einem gegenüberliegenden offenen Ende zum Einführen eines Kolbens auf. Zu dem Kolben gehört eine Kolbenstange, an deren einem Ende sich eine Griffplatte befindet, und an deren anderem Ende oder Kolbenkopf Eingriffteile zur Aufnahme einer abnehmbaren Kolbendichtung vorgesehen sind. Die Kolbendichtung ist als rohrförmiger Körper ausgebildet, dessen Außenfläche die Innenwände des Spritzengehäuses abdichtend berührt und dessen Innenfläche mit Paßteilen versehen ist, die mit den Eingriffteilen auf dem Kolbenkopf zusammenwirken, um die Kolbendichtung auf dem Kolben festzuhalten. Auf der Kolbendichtung erstreckt sich in Längsrichtung ein Schlitz, der eine Spreizung oder Aufweitung der Kolbendichtung erlaubt, so daß sie beim Zusammenbau auf den Kolbenkopf aufgesteckt werden kann, jedoch anschließend ihren normalen unaufgeweiteten Zustand wieder annimmt. Wenn die Kolbendichtung in die Mündung des Spritzengehäuses eingesetzt ist, wird sie zusammengepreßt, jedoch verbleibt auf ihrer Oberfläche an der Stelle des Schlitzes ein sehr feiner Kanal, durch den Luft aus dem Spritzengehäuse entweichen kann, wenn der Kolben bis zur Anlage der Kolbendichtung an das Medikament vorgeschoben wird.

Bei einer der bevorzugten Ausführungsformen der Spritze sind in der Innenwand des Spritzengehäuses eine oder mehrere kleine Längskanäle ausgebildet, die sich von der Mündung zu einer Stelle erstrecken, die sich etwas über dem Niveau des in dem Spritzengehäuse untergebrachten Medikamentes befindet. Dieser Kanal (oder diese Kanäle) erleichtert die Einführung des Kolbens bis zur richtigen Tiefe, insbesondere bei automatisiertem Zusammenbau.

Bei einer bevorzugten Ausführungsform des Kolbendichtungskörpers ist der Schlitz aus der im allgemeinen längsverlaufenden Richtung über ein kurzes Stück am vorderen Ende versetzt, wodurch der Dichtungskörper mit einer zusätzlichen Verriegelung versehen wird, die ihn daran hindert, sich während der Einführung des Kolbens in das Spritzengehäuse aufzudrehen, sobald er mit dem Kolbenkopf verbunden worden ist. Ferner trägt der versetzte Schlitzabschnitt am vorderen Ende der Kolbendichtung dazu bei, daß ein Ausströmen des Arzneimittels durch den von dem Schlitz gebildeten feinen Kanal verhindert wird, wenn der Kolben in das Spritzengehäuse hineingedrückt wird.

Eine weitere bevorzugte Ausführungsform der Spritze, insbesondere für eine abgemessene Dosierung des Medikamentes ist mit einem Innengewindering ausgestattet, der mit einem Gewinde auf der Kolbenstange zusammenwirkt und eine Anordnung des Ringes an jeder gewünschten Stelle auf der Kolbenstange erlaubt. Der Ring wirkt dann als Anschlag gegen die Mündung des Spritzengehäuses, um die Vorschubtiefe des Kolbens zu begrenzen. Bei Spritzen mit kleinem Durchmesser hat das Gewinde auf der Kolbenstange vorzugsweise eine größere Steigung als das Gewinde bei Spritzen mit großem Durchmesser, damit der Ring schneller in eine gewünschte Position eingestellt werden kann, insbesondere, wenn die Spritze für einen Ein-Dosis-Vorgang benutzt wird. Wenn der Benutzer den Kolben vorschiebt, bis der Ring gegen das Spritzengehäuse anliegt, kann sich der Ring beim Loslassen des Kolbens aufgrund der größeren Steigung des Gewindes zur Entspannung des Druckes zurückdrehen. Daher ist es für solche Spritzen vorteilhaft, die Mündung des Spritzengehäuses mit einer Griffplatte auszustatten, die Arretierungselemente aufweist, welche mit Arretierungselementen auf der Vorderfläche des Ringes zusammenwirken. Die zusammengreifenden Arretierungselemente verhindern diese Rückwärtsbewegung des Ringes und gewährleisten, daß die Dosis, auf die der Kolben eingestellt wurde, abgegeben wird.

Im folgenden wird die Erfindung anhand von lediglich Ausführungsbeispiele darstellenden Zeichnungen näher erläutert. Es zeigt

Figur 1 eine teilgeschnittene Seitenansicht einer erfindungsgemäßen Spritze, die mit einem Medikament vorgefüllt ist,

Figur 2 eine Teilseitenansicht des Kolbens, die die Eingriffteile an dem Kolbenkopf zeigt,

Figur 3 eine Draufsicht der Kolbendichtung,

Figur 4 eine Seitenansicht der teilweise aufgeweiteten Kolbendichtung,

Figur 5 eine Ansicht des vorderen Endes einer Ausführungsform eines Teiles zur Einstellung der Kolbenlänge,

Figur 6 eine teilgeschnittene Seitenansicht des Teiles zur Einstellung der Kolbenlänge nach Figur 5,

Figur 7 einen Längsschnitt des Spritzengehäuses der Spritze und

Figur 8 eine Draufsicht des Spritzengehäuses nach Figur 7.

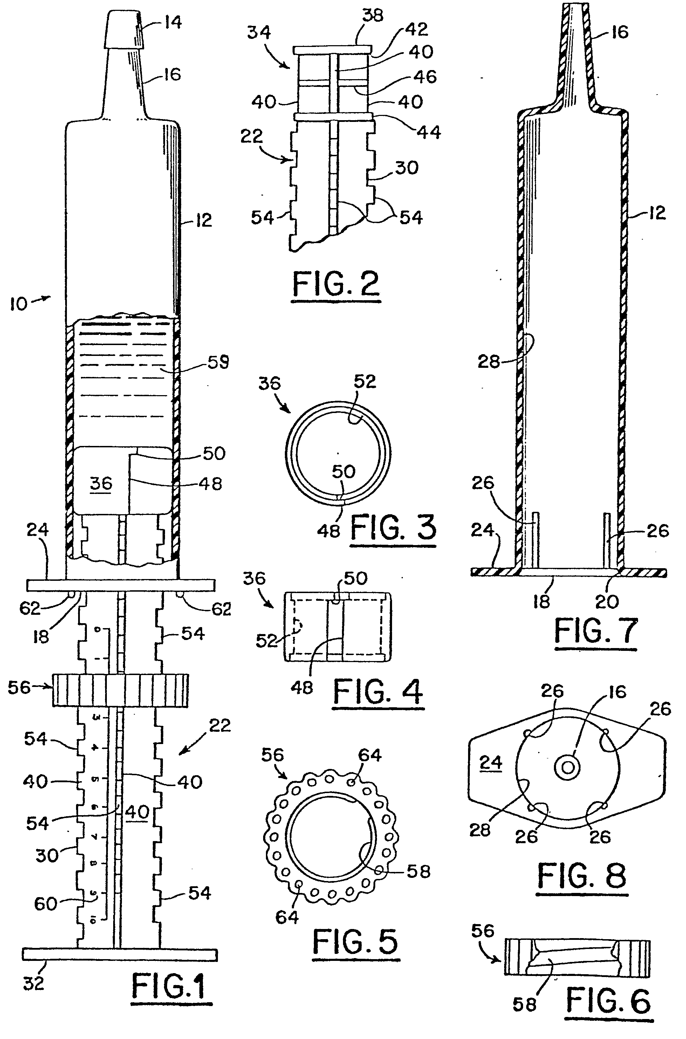

Figur 1 veranschaulicht eine Spritze 10 nach der Erfindung. Ein Spritzengehäuse 12 mit einer Kappe 14 auf dem Ausstoßende 16 weist ein offenes Ende oder eine Mündung 18 auf, die zur Erleichterung der Einführung eines Kolbens 22 mit einer leichten Abschrägung 20 (Figur 7) versehen sein kann. An der Mündung 18 des Spritzengehäuses 12 kann außerdem eine Griffplatte 24 vorgesehen sein. Bei einer bevorzugten Ausführungsform besitzt der Einlaß des Spritzengehäuses vier Kanäle 26, die sich von der Mündung 18 ein kurzes Stück über die Innenwand 28 des Spritzengehäuses erstrecken (Figuren 7 und 8) und die ebenfalls der Erleichterung der Einführung des Kolbens, insbesondere bei automatisiertem Zusammenbau, dienen.

Der Kolben 22 ist bei dieser Ausführungsform mit einer Kolbenstange 30 versehen, die an einem Ende eine Griffplatte 32 zur Fingeranlage und am vorderen Ende einen Kolbenkopf 34 trägt. Der Kolbenkopf 34, der mit einer Kolbendichtung 36 zusammenwirkt und diese festhält, besteht aus einer kreisförmigen Kopfplatte 38, die über vier mit gleichen Abständen angeordnete Radialrippen 40 übersteht, um einen vorspringenden Rand 42 zu bilden und aus einer Rückplatte 44, deren Durchmesser demjenigen der Kopfplatte 38 entspricht. Der Kolbenkopf 34 kann außerdem eine Zwischenstützplatte 46 kleineren Durchmessers aufweisen.

Die Kolbendichtung 36 ist als rohrförmiger Körper gestaltet, dessen Durchmesser etwas größer als der Durchmesser der Bohrung des Spritzengehäuses 12 ist. Die Kolbendichtung 36 ist in einer Längsrichtung geteilt, so daß ein Schlitz 48 entsteht, der ein Aufweiten der Dichtung erlaubt (Figur 4), damit sie auf den Kolbenkopf 34 aufgesetzt werden kann.

Der Schlitz 48 kann andere Formen haben und z. B. als Diagonalschlitz oder jede andere Schlitzart gestaltet sein, solange er eine Aufweitung der Dichtung ermöglicht. Vorzugsweise ist der Schlitz 48 mit einer Stufe oder irgendeiner Unregelmäßigkeit, z. B. der bei 50 gezeigten, versehen, damit eine Verriegelungszunge oder eine Struktur entsteht, die dazu beiträgt, daß ein Herausdrehen der Dichtung aus ihrer normalen Rohrform verhindert wird. Ein solcher Verriegelungsteil befindet sich am vorderen Ende der Schlitzführung. Die Dichtung 36 weist einen Zwischenabschnitt 52 auf, der dicker ist als die Wände an beiden Enden, und dieser Zwischenabschnitt paßt in die Aussparung hinein, die zwischen den Platten 38 und 44 auf dem Kolbenkopf 34 ausgebildet ist, und er verbindet die Dichtung 36 mit dem Kopf 34.

Für eine Abgabe mit Meß- oder Mehrfachdosierung ist die Kolbenstange 30 mit vier Radialrippen 40 ausgestattet, die über die gesamte Länge der Kolbenstange rechtwinklig zueinander angeordnet sind. Die Außenkanten sind in eine Vielzahl von Zähnen 54 unterteilt, und die Zähne auf der einen Rippe sind zu den Zähnen auf der benachbarten Rippe versetzt, so daß sich eine Gewindeanordnung mit einer gewissen Steigung ergibt. Ein Teil oder Ring 56 zur Einstellung der Kolbenlänge weist ein Innengewinde 58 auf, das den Ausnehmungen zwischen den Zähnen 54 angepaßt ist, so daß der Ring 56 auf der Kolbenstange 30 vorwärts oder rückwärts gedreht werden kann. Der Ring 56 wird zuerst auf die Kolbenstange 30 aufgeschraubt, bevor die Kolbendichtung 36 auf den Kolbenkopf 34 aufgesetzt wird.

Nachdem in das Spritzengehäuse 12 eine angemessene Menge einer gewünschten Arzneimittelzubereitung 59, z. B. eines pastenförmigen Wurmmittels, eingefüllt worden ist, wird der Kolben in die Mündung des Spritzengehäuses eingesetzt, und bei seiner Niederdrückung auf das Niveau des Medikamentes entweicht Luft in dem Spritzengehäuse zunächst durch die Kanäle 26, bis die Dichtung 36 sich hinter ihnen befindet, und an dieser Stelle kann die Restluft durch den von dem Schlitz 48 gebildeten Kanal strömen und aus der Spritze austreten. Der Ring 56 wird dann auf eine durch Markierungen 60 bezeichnete Stelle gedreht und nach Entfernung der Kappe 14 wird die gewünschte Dosis des Medikamentes ausgespritzt, indem der Kolben vorgeschoben wird, bis der Ring 56 gegen die Griffplatte 24 an dem Spritzengehäuse zur Anlage kommt.

Spritzen, deren Spritzengehäuse einen ziemlich kleinen Durchmesser aufweisen, und bei denen deshalb die Kolbenstange des Kolbens recht schmal ist, müßten notwendigerweise entweder kleinere Zähne 54 aufweisen, um die gleiche Steigung wie bei längerem Kolben zu erzielen oder die Steigung wäre bei Verwendung von Zähnen gleicher Größe viel größer. Die kleineren Ein-Dosis-Spritzen werden häufig benutzt, um pastenförmige Wurmmittel in das Maul eines Pferdes einzubringen, und es ist für einen Tierarzt lästig, einen Ring auf dem Kolben mit sehr vielen Umdrehungen verstellen zu müssen, bis der Ring die richtige Einstellung erreicht hat. Deshalb wird eine Gewinde-Kolbenstange mit einer steileren Steigung bevorzugt. Wenn der Kolben gegen die Paste in einer solchen Spritze gedrückt und der Fingerdruck auf die Griffplatte (32) nicht aufrechterhalten wird, preßt der Gegendruck gegen den Ring und verursacht manchmal seine Rückwärtsdrehung. Man kann dann nicht sicher sein, daß die richtige Dosis appliziert wurde. Zur Umgehung dieses Problems sind an der Griffplatte 24 des Spritzengehäuses 12 und an dem Ring 56 Arretierungselemente angebracht. Bei dem in den Figuren 1 und 5 dargestellten Ausführungsbeispiel haben diese Arretierungselemente die Form von zwei Vorsprüngen oder Nasen 62, von denen je eine auf den beiden Seiten der Mündung 18 von der Griffplatte 24 vorsteht, und es sind eine Reihe kleiner Vertiefungen 64 umfangsmäßig auf der Vorderfläche des Ringes 56 angeordnet. Wenn die Nasen 62 in zwei Vertiefungen 64 eingreifen, ist der Ring in seiner Stellung gesichert und kann sich unter Druck nicht rückwärts bewegen.

Da zahlreiche Abwandlungen im Rahmen der Erfindung möglich sind, sollen die beschriebenen Beispiele lediglich der Veranschaulichung dienen.

**Ansprüche**

1. Spritze (10) mit einem Spritzengehäuse (12), an dem ein Ausstoßende (16) und ein offenes, gegenüberliegendes Ende (18) zur Aufnahme eines Kolbens (22) ausgebildet sind, wobei zu dem Kolben (22) eine Kolbenstange (30) gehört, an deren einem Ende sich eine Griffplatte (32) befindet und an deren anderem Ende ein Kolbenkopf (34) mit einer daran befestigten abnehmbaren Kolbendichtung (36) vorgesehen sind, welche aus einem im wesentlichen rohrförmigen Körper mit einem vorderen und einem hinteren Ende besteht, dessen Außenfläche auf der Innenfläche des Spritzengehäuses (12) verschiebbar ist und der auf seiner Innenseite Paßteile (52) aufweist, die mit Eingriffteilen (38, 40, 42, 44, 46) auf dem Kolbenkopf (34) zusammenwirken, dadurch gekennzeichnet, daß der rohrförmige Körper zur Bildung eines einzigen Schlitzes (48) in einer im wesentlichen längsverlaufenden Richtung geteilt ist, damit er zum Zusammensetzen mit den Eingriffteilen aufweitbar ist, jedoch anschließend seinen normalen unaufgeweiteten Zustand wieder annimmt, wenn er mit den Eingriffteilen vollständig zusammengreift.

2. Spritze nach Anspruch 1, dadurch gekennzeichnet, daß der Schlitz (48) in der Kolbendichtung (36) an seinem vorderen Ende eine Verriegelung aufweist, die aus einer Unregelmäßigkeit (50) in dem Schlitz (48) in einer zu der allgemeinen Längsrichtung des Schlitzes (48) entgegengesetzten Richtung besteht.

3. Spritze nach Anspruch 2, dadurch gekennzeichnet, daß auf die Kolbenstange (30) ein drehbarer Teil (56) zur Einstellung der Länge des Kolbens (22) aufgeschraubt ist, der durch Zusammenwirken mit Gewindeteilen (54) auf der Kolbenstange (30) auf dieser hin- und herbewegbar ist, und daß der Durchmesser des drehbaren Teiles (56) größer ist als derjenige des Spritzengehäuses (12), damit er an diesem zur Anlage kommt und den Kolben (22) an einer vorbestimmten Stelle innerhalb des Spritzengehäuses (12) festlegt.

4. Spritze nach Anspruch 3, dadurch gekennzeichnet, daß auf der Innenfläche des Spritzengehäuses (12) wenigstens eine Nut (26) ausgebildet ist, die sich von dem offenen Ende (18) längsverlaufend über ein Teilstück in das Spritzengehäuse (12) erstreckt, das etwa der Länge der Kolbendichtung (36) entspricht.

5. Spritze nach Anspruch 4, dadurch gekennzeichnet, daß vier Nuten (26) mit im wesentlichen gleichen Abständen angeordnet sind.

6. Spritze nach Anspruch 3, dadurch gekennzeichnet, daß an dem offenen Ende (18) des Spritzengehäuses (12) eine Griffplatte (24) angeordnet ist, mit der Arretierungselemente (62) einstückig ausgebildet sind, und daß der drehbare Teil (56) einen Ring mit Innengewinde (58) aufweist, auf dessen Vorderfläche Arretierungselemente (64) ausgebildet sind, wobei die jeweiligen Arretierungselemente (62 bzw. 64) des drehbaren Teiles (56) und der Griffplatte (24) des Spritzengehäuses (12) ineinandergreifen und eine Bewegung des drehbaren Teiles (56) verhindern, wenn Druck auf den Kolben (22) ausgeübt wird.

7. Spritze nach Anspruch 6, dadurch gekennzeichnet, daß die Arretierungselemente an der Griffplatte (24) aus zwei Vorsprüngen (62) bestehen, und daß die Arretierungselemente an dem drehbaren Teil (56) aus mehreren Vertiefungen (64) gebildet sind, die in der Vorderfläche des Ringes umfangsmäßig angeordnet und zur Aufnahme der Vorsprünge (62) geeignet sind.

## Claims

1. A syringe (10) with a syringe barrel (12) on which a discharge end (16) and an open opposite end (18) for receiving a plunger (22) are formed, the plunger (22) having a plunger shaft (30) at the one end of which there is a finger-engaging ledge (32) and at the other end of which there is a plunger head (34) with a plunger seal (36) which is attached to the plunger head and is removable and which consists of a generally tubular element with a front and rear end, the external surface of which is able to be moved along the internal surface of the syringe barrel (12) and which has fitment sections (52) on its interior which coact with engagement sections (38, 40, 42, 44, 46) on the plunger head (34), characterised in that the tubular element is severed along a generally longitudinal direction for the formation of a single slit (48), so that it can be expanded for coacting with the engagement sections, but then reassumes its normal unexpanded condition when it fully engages with the engagement sections.

2. The syringe according to Claim 1, characterised in that the slit (48) in the plunger seal (36) has locking means at the front end consisting of an irregularity (50) in the slit (48) in a direction opposed to the generally longitudinal direction of the slit (48).

3. The syringe according to Claim 2, characterised in that a rotatable member (56) is screwed on to the plunger shaft (30) for adjusting the length of the plunger (22) which is able to be moved in either direction on the shaft by coacting with threaded sections (54) on the plunger shaft (30) and in that the diameter of the rotatable member (56) is larger than that of the syringe barrel (12), so that it is arrested by this and fixes the plunger (22) at a preselected location within the syringe barrel (12).

4. The syringe according to Claim 3, characterised in that at least one groove (26) is formed on the interior surface of the syringe barrel (12), which groove extends longitudinally from the open end (18) over a section of the syringe barrel (12) which approximately corresponds to the length of the plunger seal (36).

5. The syringe according to Claim 4, characterised in that there are four grooves (26) substantially equally spaced.

6. The syringe according to Claim 3, characterised in that a finger-engaging ledge (24) is arranged at the open end (18) of the syringe barrel (12) on which ledge arresting elements (62) are integrally formed, and in that the rotatable section (56) has a ring with an internal thread (58) on the front surface of which arresting elements (64) are formed, whereby the respective arresting elements (62 and 64) of the rotatable section (56) and the finger-engaging ledge (24) of the syringe barrel (12) interlock and prevent movement of the rotatable section (56) when pressure is exerted on the plunger (22).

7. The syringe according to Claim 6, characterised in that the arresting elements on the finger-engaging ledge (24) consist of two projections (62) and in that the arresting elements on the rotatable section (56) are formed of a plurality of cavities (64) which are arranged circumferentially in the front surface of the ring and are suitable for receiving the projections (62).

## Revendications

1. Seringue (10) comprenant un corps de seringue (12) sur lequel sont formées une extrémité de sortie (16) et une extrémité ouverte opposée (18) destinée à recevoir un piston (22) cependant qu'au piston (22) appartient une tige de piston (30) à une extrémité de laquelle se trouve une plaque de prise (32) et à l'autre extrémité de laquelle se trouve une tête de piston (3) munie d'un joint de piston amovible (36) fixé à cette tête, qui est composé d'un corps sensiblement tubulaire muni d'une extrémité avant et d'une extrémité arrière, dont la surface externe peut coulisser sur la surface interne du corps (12) de la seringue et qui présente, sur son côté interne, des éléments ajustés (52) qui coopèrent avec des éléments de prise (38, 40, 42, 44, 46) prévus sur la tête (34) du piston, caractérisée en ce que le corps tubulaire est divisé, dans une direction sensiblement longitudinale, pour former une fente unique (48) afin qu'on puisse l'élargir pour l'assemblage avec les parties de prise mais que, ensuite, il reprenne son état normal non élargi lorsqu'il est entièrement en prise avec les parties de prise.

2. Seringue suivant la revendication 1, caractérisée en ce que la fente (48) prévue dans le joint (36) du piston présente à son extrémité avant un verrouillage qui est constituée par une irrégularité (50) prévue dans la fente (48), dans une direction opposée à la direction longitudinale générale de la fente (48).

3. Seringue suivant la revendication 2, caractérisée en ce que, sur la tige de piston (30) est vissée une pièce tournante (56) pour le réglage de la longueur du piston (22), pièce qui peut, par coopération avec des éléments de filetage (54) prévus sur la tige (30) du piston, se déplacer dans les deux sens sur celle-ci et en ce que le diamètre de la pièce tournante (56) est plus grand que celui du corps (12) de la seringue afin qu'il entre en contact avec ce corps et bloque le piston (22) en une zone prédéterminée à l'intérieur du corps (12) de la seringue.

4. Seringue suivant la revendication 3, caractérisée en ce que, sur la surface interne du corps (12) de la seringue, est formée au moins une rainure (12) qui, à partir de l'extrémité ouverte (18) s'étend, longitudinalement, sur une longueur partielle dans le corps (12) de la seringue qui correspond à peu près à la longueur du joint (36) du piston.

5. Seringue suivant la revendication 4, caractérisée en ce que quatre rainures (26) sont disposées à des écartements sensiblement égaux.

6. Seringue suivant la revendication 3, caractérisée en ce qu'à l'extrémité ouverte (18) du corps (12) de la seringue est disposée une plaque de prise (24) avec laquelle des éléments d'arrêt (62) sont formés d'une seule pièce et en ce que la pièce tournante (56) présente une bague munie d'un filetage intérieur (58) et sur la surface avant de laquelle sont formés des éléments d'arrêt (64), les éléments d'arrêt (62 ou 64) respectivement de la pièce tournante (56) et de la plaque de prise (24) du corps (12) de la seringue s'engageant les uns dans les autres et empêchant un mouvement de la pièce tournante (56) lorsqu'une pression est exercée sur le piston (22).

7. Seringue suivant la revendication 6, caractérisée en ce que les éléments d'arrêt portés par la plaque de prise (24) sont constitués par deux saillies (62) et en ce que les éléments d'arrêt prévus sur la pièce tournante (56) sont composés de plusieurs évidements (64) qui sont disposés circonférentiellement dans la surface avant de la bague et appropriés pour recevoir les saillies (62).

FIG. 2

FIG. 3

FIG. 4

FIG. 5

FIG. 6

FIG. 7

FIG. 8

FIG.1